(19) Europäisches Patentamt / European Patent Office / Office européen des brevets

(11) **EP 2 882 332 B1**

(12) **FASCICULE DE BREVET EUROPEEN**

(45) Date de publication et mention
de la délivrance du brevet:
**04.04.2018 Bulletin 2018/14**

(21) Numéro de dépôt: **13758917.2**

(22) Date de dépôt: **25.07.2013**

(51) Int Cl.:
**A61B 3/09** *(2006.01)*     **A61B 3/00** *(2006.01)*

(86) Numéro de dépôt international:
**PCT/FR2013/051799**

(87) Numéro de publication internationale:
**WO 2014/023889 (13.02.2014 Gazette 2014/07)**

(54) **PROCÉDÉ ET DISPOSITIF DE DÉPISTAGE D'UN ÉTAT DE FATIGUE OPHTALMIQUE D'UN INDIVIDU**

VERFAHREN UND VORRICHTUNG ZUR PRÜFUNG DES ZUSTANDS DER OPHTHALMISCHEN ERMÜDUNG EINES INDIVIDUUMS

METHOD AND DEVICE FOR SCREENING A STATE OF OPHTHALMIC FATIGUE OF AN INDIVIDUAL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.08.2012 FR 1202219**

(43) Date de publication de la demande:
**17.06.2015 Bulletin 2015/25**

(73) Titulaire: **Essilor International
94220 Charenton-le-Pont (FR)**

(72) Inventeur: **DROBE, Björn
94220 Charenton le Pont (FR)**

(74) Mandataire: **Jacobacci Coralis Harle
32, rue de l'Arcade
75008 Paris (FR)**

(56) Documents cités:
**FR-A1- 2 861 280     FR-A1- 2 880 789
US-A- 698 833     US-A1- 2006 103 808
US-B1- 7 341 350**

**Description**

DOMAINE TECHNIQUE AUQUEL SE RAPPORTE L'INVENTION

La présente invention concerne de manière générale les méthodes de diagnostic de l'accommodation ophtalmique des individus.

Elle concerne plus particulièrement un procédé de dépistage d'un état de fatigue ophtalmique d'un individu, comportant des étapes :

a1) de positionnement d'une cible relativement à l'individu de telle manière que la cible soit visible par au moins un des deux yeux de l'individu,
a2) d'appréciation par l'individu de la netteté de la cible, et
a3) de déduction d'un état de fatigue ophtalmique de l'individu selon que la cible est vue par l'individu de manière nette ou floue.

Elle concerne également un dispositif de dépistage d'un état de fatigue ophtalmique d'un individu, comportant :

- un support comportant une partie d'appui à appliquer contre le visage d'un individu et une glissière,
- une cible montée mobile en coulissement sur la glissière du support, et
- au moins une graduation située le long de la glissière pour repérer la position de la cible sur la glissière.

ARRIERE-PLAN TECHNOLOGIQUE

L'accommodation est définie comme l'action automatique et involontaire des muscles ciliaires pour déformer l'oeil de manière à ce qu'il puisse voir de manière nette des objets situés à des distances variées.

Plus précisément, durant l'accommodation, la courbure du cristallin change sous l'influence des muscles ciliaires, ce qui permet la formation d'images claires sur la rétine. Le cristallin s'aplatit pour une vision nette des objets éloignés et s'arrondit pour voir les objets rapprochés.

La distance minimale d'accommodation d'un individu donné est alors définie comme la distance à partir de laquelle cet individu peut voir un objet de manière parfaitement nette. En deçà de cette distance minimale d'accommodation, les muscles ciliaires ne permettent pas d'arrondir suffisamment le cristallin pour permettre la formation d'images claires sur la rétine.

On sait que cette distance minimale d'accommodation augmente avec l'âge, et qu'elle augmente plus ou moins rapidement d'un individu à l'autre. Elle augmente également lorsque l'individu est fatigué.

Il est alors connu de mesurer cette distance minimale d'accommodation en utilisant une règle ophtalmique, plus connue sous l'expression anglaise « RAF rule » (pour « Royal Air Force rule»).

Cette règle ophtalmique comporte, d'une part, une tige métallique graduée en dioptries et en mètres, dont une extrémité forme une fourche à appliquer contre le visage du porteur, et, d'autre part, une cible montée mobile en translation sur la tige métallique.

Le procédé d'utilisation de cette règle consiste à positionner la cible sur la tige à l'opposé de la fourche, puis à appliquer cette fourche sur le visage de l'individu en maintenant la tige métallique dans l'axe des yeux de l'individu, et enfin à rapprocher progressivement la cible jusqu'à une position limite au niveau de laquelle l'individu ne voit plus cette cible de manière nette.

La distance minimale d'accommodation peut alors directement être lue sur la graduation prévue à cet effet.

La demanderesse a toutefois constaté que l'utilisation de cette règle ophtalmique aboutit généralement à des mesures surestimées, en ce sens que les distances minimales d'accommodation mesurées sont généralement plus faibles que les distances minimales d'accommodation réelles des porteurs.

Il s'en suit des erreurs de prescription, en ce sens qu'aucune paire de lunettes n'est prescrite à certaines personnes qui nécessiterait pourtant des lunettes dites antifatigue.

On connaît par ailleurs du document US7341350 un appareil capable de déterminer une amplitude d'accommodation et qui comprend à cet effet un cible déplaçable sur une glissière le long de laquelle sont marquées des distances.

On connaît aussi du document US698833 un appareil de détermination de la plage d'accommodation d'un oeil d'un individu. Cet a pareil comporte à cet effet un châssis qui comprend un anneau à antérieur duquel est montée une lentille, un manchon qui s'étend en longueur à partir de l'anneau et dont l'extrémité libre forme une zone d'appui pour le visage de l'individu, une règle qui est fixée au châssis et qui présente une graduation, et une cible qui est montée mobile sur la règle. Il est noté que la graduation out faire apparaître des âges.

OBJET DE L'INVENTION

Afin de remédier à l'inconvénient précité de l'état de la technique, la présente invention propose un nouveau procédé de diagnostic de l'état de fatigue ophtalmique d'un individu, permettant de déterminer avec précision si cet individu nécessite ou non des lunettes antifatigue ou des soins particuliers (conseils sur l'ergonomie de l'éclairage, larmes artificielles pour lutter contre la sécheresse oculaire, ...).

Plus particulièrement, on propose selon l'invention un procédé de dépistage tel que défini dans l'introduction, dans lequel il est prévu, préalablement à l'étape a2), une étape a0) de réglage de la taille de la cible et/ou de la position de la cible relativement à l'individu, en fonction de l'âge de l'individu.

La demanderesse a constaté que les erreurs de mesure à l'aide d'une règle du type « RAF rule » résultent du rapprochement progressif de la cible vers les yeux de l'individu, qui permet aux muscles ciliaires de se contracter progressivement jusqu'à un seuil au-delà duquel ils sont capables de se contracter instantanément.

La présente invention consiste alors non plus à rapprocher progressivement la cible de l'individu, mais plutôt à régler la position et/ou la taille de la cible à une valeur donnée, puis, une fois que la cible est réglée, à demander à l'individu de regarder la cible afin qu'il puisse déterminer s'il la voit de manière nette.

La taille et/ou la position de la cible est alors prédéterminée en fonction de l'âge de l'individu, grâce à des données issues d'études statistiques qui permettent de déterminer la distance minimale d'accommodation qu'un individu est sensé présenter à un âge donné, s'il ne souffre d'aucun problème ophtalmique particulier.

On rappellera par ailleurs ici quelques généralités au sujet de la fatigue ophtalmique.

La distance minimale d'accommodation d'un individu évolue naturellement avec l'âge d'un individu. Cette évolution ne constitue pas une fatigue ophtalmique puisqu'elle est durable est irréversible.

La fatigue ophtalmique est au contraire un état passager et réversible de fatigue des muscles ciliaires de l'individu. C'est un état normal, qui atteint des individus sains après que leurs muscles ciliaires ont été fortement sollicités.

Ce que l'invention cherche ici à déterminer ne concerne alors pas la composante irréversible des problèmes visuels de l'individu, mais plutôt la composante occasionnelle et non pathologique de ces problèmes.

Lors de la mise en oeuvre de l'invention, il n'est d'ailleurs pas question de comparer des valeurs mesurées sur l'individu avec celles d'un individu sain, mais plutôt des valeurs mesurées sur l'individu dont les muscles ciliaires sont fatigués avec celles qui seraient mesurées sur le même individu, par exemple au réveil, lorsque ses muscles ciliaires sont reposés.

Avantageusement, si, à l'étape a2), la cible est vue floue par l'individu, il est prévu suite à l'étape a3) des étapes :

b1) d'agrandissement de la taille de la cible et/ou d'éloignement de la cible relativement à l'individu d'une valeur prédéterminée,

b2) d'appréciation par l'individu de la netteté de la cible, et

b3) si, à l'étape b2), l'individu voit la cible de manière plus nette, de déduction d'un besoin d'une correction antifatigue.

La correction de la taille et/ou de la position de la cible permet alors de vérifier si le défaut d'accommodation constaté provient bien d'un problème de fatigue ophtalmique auquel une paire de lunettes antifatigue peut remédier, ou bien s'il s'agit d'un problème plus sérieux (auquel cas l'individu devra subir des examens complémentaires).

D'autres caractéristiques avantageuses et non limitatives du procédé conforme à l'invention sont les suivantes :

- au cours de l'étape a0), seule la distance entre la cible et l'individu est réglée ;
- l'étape b1) consiste à insérer au moins une lentille de correction entre l'oeil de l'individu et la cible ;
- la lentille de correction présente une puissance sphérique non nulle et/ou une puissance cylindrique non nulle et/ou une puissance prismatique non nulle et/ou un revêtement antireflet et/ou un revêtement teinté, et/ou est formée par une lentille à profondeur de champ ;
- à l'étape a0), la distance entre la cible et l'individu est réglée en fonction également du moment de la journée auquel est effectué le dépistage, et/ou de l'origine géographique de l'individu et/ou de la netteté de la cible et/ou du contraste de la cible ;
- au cours de l'étape a0), seule la taille de la cible est réglée ;
- l'étape b1) consiste à agrandir la taille de la cible ;
- préalablement à l'étape a2), on équipe l'individu de son équipement de correction habituel (par exemple sa paire de lunettes de correction habituelle ou ses lentilles de contact habituelles) ;
- les étapes a1) et a2) sont réalisées simultanément ou successivement sur les deux yeux de l'individu ; et
- suite à l'étape a3), il est prévu une étape c1) de mémorisation et de transmission à distance, via un réseau informatique, de l'état de fatigue ophtalmique déduit à l'étape a3).

L'invention propose également un dispositif de dépistage tel que défini en introduction, dans lequel ladite graduation fait apparaître des âges.

D'autres caractéristiques avantageuses et non limitatives du dispositif conforme à l'invention sont les suivantes :

- le support comporte des moyens de positionnement d'une lentille de correction, situés entre la partie d'appui et la cible ;
- il est prévu au moins deux graduations faisant apparaître des âges et il est prévu des moyens de positionnement d'une lentille de changement d'échelle ; et
- la cible présente au moins une lettre ou une image.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est une vue schématique en perspective d'un premier mode de réalisation d'un dispositif de dépistage selon l'invention ;
- la figure 2 est une vue schématique en perspective de la cible du dispositif de dépistage de la figure 1 ;
- la figure 3 est une vue schématique en perspective d'un second mode de réalisation du dispositif de dépistage selon l'invention ;
- la figure 4 est une vue schématique en perspective d'un troisième mode de réalisation du dispositif de dépistage selon l'invention ;
- la figure 5 est une vue schématique en perspective d'un quatrième mode de réalisation du dispositif de dépistage, adapté à mettre en oeuvre un procédé de dépistage selon l'invention ; et
- la figure 6 est un tableau illustrant les variations de la distance minimale d'accommodation en fonction de l'âge des individus et de la puissance de la lentille de mise à l'échelle.

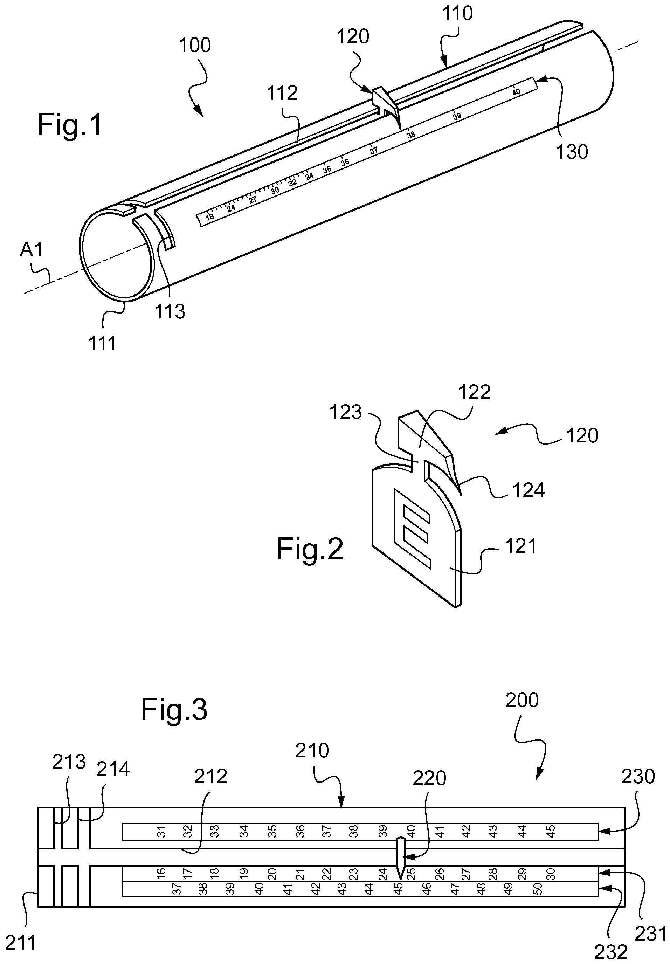

Sur les figures 1, 3, 4 et 5, on a représenté quatre modes de réalisation d'un dispositif de dépistage 100 ; 200 ; 300 ; 400 d'un état de fatigue ophtalmique d'un individu.

La fatigue ophtalmique (ou « fatigue visuelle») est un effet physiologique réversible résultant de sollicitations excessives des muscles oculaires et de la rétine, pour tenter de conserver une image nette par des ajustements inefficaces. Elle s'accompagne d'une réduction de la capacité nécessaire à la réalisation d'une tâche visuelle et d'une modification de la stratégie d'accomplissement de cette tâche ; elle constitue un signal d'alarme qu'il convient de traiter.

La fatigue ophtalmique peut se traduire non seulement par des symptômes subjectifs et des modifications physiologiques, mais aussi par une baisse de la performance visuelle.

La fatigue ophtalmique est plus précisément étudiée dans le document « La fatigue visuelle » émis par le service de physiologie environnementale de l'INRS (Institut National de Recherche et de Sécurité) en mars 1992.

Dans chacun de ces quatre modes de réalisation, le dispositif de dépistage 100 ; 200 ; 300 ; 400 est plus précisément prévu pour faciliter la tâche d'un opticien qui consiste à déterminer si un patient présente ou non une bonne vision de près et si, dès lors, il nécessite ou non une paire de lunettes dite antifatigue.

Pour cela, le dispositif de dépistage 100 ; 200 ; 300 ; 400 est prévu pour déterminer si, compte tenu de l'âge du patient, chacun des deux yeux du patient présente une « distance minimale d'accommodation » satisfaisante.

Par « distance minimale d'accommodation », on entend la distance minimale à laquelle un individu peut voir un objet de manière nette.

Dans les trois premiers modes de réalisation respectivement représentés sur les figures 1, 3 et 4, le dispositif de dépistage 100 ; 200 ; 300 comprend :

- un support 110 ; 210 ; 310 comportant une partie d'appui 111 ; 211 ; 311 à appliquer contre le visage ou les lunettes du patient, et une glissière 112 ; 212 ; 312,
- une cible 120 ; 220; 320 montée mobile en coulissement sur la glissière 112 ; 212 ; 312 du support 110 ; 210 ; 310, et
- au moins une graduation 130 ; 230, 231, 232 ; 330, 331 située le long de la glissière 112 ; 212 ; 312 pour repérer la position de la cible 120 ; 220 ; 320 sur la glissière 112 ; 212 ; 312.

Selon une caractéristique particulièrement avantageuse du dispositif de dépistage selon l'invention, cette graduation 130; 230, 231, 232 ; 330, 331 fait apparaître des âges.

Selon une caractéristique avantageuse de ce dispositif de dépistage, le support 110 ; 210 ; 310 comporte en outre des moyens de positionnement 113 ; 213 ; 313 d'une lentille de correction entre la partie d'appui 111 ; 211 ; 311 et la cible 120 ; 220 ; 320, pour faciliter au besoin la vision de la cible.

Ces moyens de positionnement 113 ; 213 ; 313 sont donc conçus pour recevoir au besoin une lentille de correction, de manière que cette dernière reste amovible.

Ils pourront donc se présenter sous diverses formes, pour autant qu'ils permettent de positionner et d'extraire la lentille de correction manuellement, sans l'aide d'outil.

Les différents modes de réalisation du dispositif de dépistage seront plus précisément décrits dans la suite de cet exposé.

Sur la figure 1, on a représenté le premier mode de réalisation du dispositif de dépistage 100.

Dans ce mode de réalisation, le support 110 se présente sous la forme d'un tube sensiblement cylindrique de révolution autour d'un axe longitudinal A1.

Ce support tubulaire 110 présente ici une longueur de 30 centimètres, un diamètre intérieur de 5 centimètres, et un diamètre extérieur de 5,4 centimètres.

Il est réalisé d'une seule pièce par moulage d'une matière plastique transparente.

La partie d'appui 111 de ce support tubulaire 110, qui est destinée à être appliquée contre le visage du patient ou contre la paire de lunettes que le patient porte, est alors formée par l'une des extrémités circulaires de ce support tubulaire 110.

La glissière 112 de ce support tubulaire 110, dans laquelle coulisse la cible 120, est quant à elle formée par une fente longitudinale rectiligne qui s'étend sur l'ensemble de la longueur du support tubulaire 110, parallèlement à l'axe longitudinal A1, et qui présente ici une largeur de 5 millimètres.

Telle que représentée sur la figure 1, la graduation 130 sur laquelle apparaissent des âges est imprimée sur un autocollant rectangulaire, collé parallèlement à la glissière 112, le long de celle-ci.

Cet autocollant porte, à la manière d'un réglet, deux types de marquages. Il porte tout d'abord, le long de son bord qui longe la glissière 112, vingt-trois tirets courts, parallèles et espacés deux à deux, d'axes orthogonaux à l'axe longitudinal A1. Il porte par ailleurs, en face d'une partie au moins de ces tirets, des chiffres indiquant des âges.

Chacun des tirets est associé à un âge donné, compris entre 18 et 40 ans. L'espacement entre les tirets croît depuis la partie d'appui 111 jusqu'à l'extrémité opposée du support tubulaire 110.

Chacun des âges notés sur la graduation 130 indique l'âge associé au tiret en face duquel il se trouve. Les âges notés sur la graduation 130 vont croissant depuis la partie d'appui 111 jusqu'à l'extrémité opposée du support tubulaire 110.

Chaque tiret est alors situé à une distance de la partie d'appui 111 du support tubulaire 110 qui est sensiblement égale à la distance minimale d'accommodation qu'un individu est sensé présenter à un âge donné (celui auquel ce tiret est associé), s'il ne souffre d'aucun problème ophtalmique particulier.

Cette distance pourrait être obtenue par une étude statistique effectuée sur un échantillon représentatif d'individus ne souffrant d'aucun problème ophtalmique particulier.

Ici, cette distance notée $D_{âge}$ est calculée au moyen de la formule mathématique suivante :

$$D_{âge} = 1 \: / \: (A_{âge} - G),$$

avec :

$A_{âge}$ la distance minimale d'accommodation (exprimée en dioptries) attendue pour un individu d'âge donné, et
G un coefficient correcteur.

L'amplitude minimale d'accommodation $A_{âge}$ (ou « punctum proximum d'accomodation »), exprimée en dioptries, est calculée suivant la formule de Hofsetter, selon laquelle :

$$A_{âge} = 18,5 - 1/3 \: . \: âge$$

Le coefficient correcteur G est quant à lui ici choisi égal à 0.

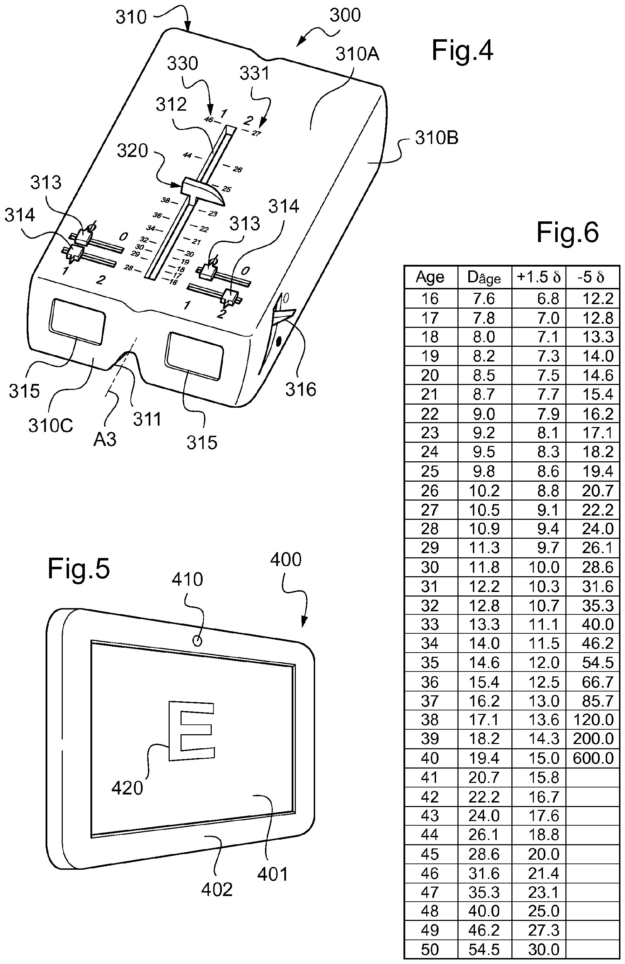

Le résultat obtenu est fourni, en millimètres, sur la seconde colonne du tableau de la figure 6.

Ce coefficient correcteur G, qui sert à moduler le seuil à partir duquel on estime qu'il y a fatigue ophtalmique, pourrait en variante être choisi non nul, par exemple pour tenir compte d'un manque de netteté ou d'un faible contraste de la cible 120.

Il pourrait également être choisi non nul pour tenir compte de la zone géographique dans laquelle travaille l'opticien. On a en effet constaté que la distance minimale d'accommodation d'un individu ne souffrant d'aucun problème ophtalmique particulier varie d'une zone géographique à l'autre.

La cible 120, qui est prévue pour coulisser le long de la glissière 112, est quant à elle plus précisément représentée sur la figure 2.

Elle présente une partie de lecture 121, sur laquelle est représenté un motif quelconque, une partie de préhension 122 facilement manipulable par un opticien pour faire coulisser la cible 120 le long de la glissière 112, et une partie de liaison 123 qui relie la partie de lecture 121 à la partie de préhension 122.

La partie de lecture 121 est logée à l'intérieur du support tubulaire 110 et elle porte un motif facilement reconnaissable par le patient.

Cette partie de lecture 121 se présente ici sous la forme d'une plaquette carrée, d'environ 2 centimètres de côtés, dont les deux faces principales sont orientées orthogonalement à l'axe longitudinal A1.

Sa face principale orientée vers la partie d'appui 111 est celle sur laquelle est imprimé le motif.

Ce motif est ici formé par une lettre (ici un « E »). Il pourrait en variante être formé par un dessin tel qu'un animal, ou par un ensemble de lettres de tailles variables.

Ce motif présente ici une couleur noire et est dessiné sur un fond blanc. En variante, on pourrait prévoir que le fond soit d'une couleur différente, par exemple vert ou rouge, et que le motif présente une autre couleur.

La partie de préhension 122 est quant à elle située à l'extérieur du support tubulaire 110.

Elle se présente sous la forme d'une plaquette rectangulaire, qui est ici coplanaire avec la partie de lecture 121. En variante, elle pourrait être décalée par rapport à la partie de lecture 121, pour assurer une meilleur préhension.

Elle porte ici à une de ses extrémités une pointe 124 qui permet de lire précisément sur la graduation 130 l'âge correspondant à la position de la cible 120 le long de la glissière 112.

La partie de liaison 123 présente quant à elle une largeur égale, au jeu près, à la largeur de la glissière 112 et une hauteur égale, au jeu près, à l'épaisseur du support tubulaire 110, pour permettre de guider la cible 120 en translation le long de cette glissière 112.

Comme le montre la figure 1, les moyens de positionnement, qui permettent d'interposer une lentille de correction entre la partie d'appui 111 du support tubulaire 110 et la cible 120, sont ici formés par une fente transversale 113.

Cette fente transversale 113 s'étend sur la moitié de la périphérie du support tubulaire 110, de part et d'autre de la glissière 112. Elle permet ainsi d'accueillir au besoin une quelconque lentille de correction, lorsque l'opticien le souhaite. L'utilisation de cette lentille sera décrite infra.

Le dépistage de l'éventuel état de fatigue ophtalmique du patient au moyen de ce dispositif de dépistage 100 est alors opéré de la manière suivante.

Au préalable, l'opticien interroge le patient pour connaître son âge.

Il lui demande par ailleurs de s'équiper de son équipement de correction habituel (par exemple de ses lunettes de vue ou de ses lentilles de contact).

L'opticien règle alors la position de la cible 120 dans la glissière 112 de telle manière que sa pointe 124 vise, sur la graduation 130, l'âge indiqué par le patient.

L'opticien demande ensuite au patient de fermer l'un de ses deux yeux, par exemple l'oeil gauche.

Il positionne alors le dispositif de dépistage 100 dans l'axe de l'oeil gauche du patient, la partie d'appui 111 au contact du visage ou des lunettes du patient, de telle manière que lorsque celui-ci ouvre son oeil gauche, il puisse voir la partie de lecture 121 de la cible 120.

L'opticien demande alors au patient de juger de la netteté de la lettre imprimée sur la cible 120.

Selon que le patient y parvient ou non, l'opticien en déduit que le patient présente ou pas un état de fatigue ophtalmique.

Plus précisément, si le patient juge la lettre nette (par exemple s'il parvient à lire la lettre « E »), l'opticien en déduit que l'oeil correspondant de l'individu ne présente pas d'état de fatigue. L'opticien en déduit donc que le patient ne nécessite pas, au moins pour son oeil gauche, de paire de lunettes antifatigue.

Au contraire, si le patient juge la lettre peu nette (par exemple s'il ne parvient pas à lire la lettre « E »), l'opticien procède à un examen complémentaire afin de vérifier que seul un état de fatigue affecte l'oeil gauche du porteur (et que ce problème de vision n'est pas plus sérieux).

Pour cela, l'opticien place une lentille de correction au travers de la fente transversale 113 du support tubulaire 110, dont la puissance est telle qu'elle permet de compenser un léger défaut d'accommodation de l'oeil, en éloignant et en agrandissant artificiellement la taille de la lettre « E » vue par le patient.

La lentille de correction utilisée sera de préférence une simple lentille convergente, dont la puissance sphérique sera strictement positive (par exemple égale à 0,6 dioptrie). Elle pourra également être formée par une lentille à profondeur de champ.

En variante, on pourra prévoir que cette lentille de correction présente une puissance cylindrique non nulle (seule ou combinée avec une puissance sphérique), pour corriger une éventuelle fatigue cylindrique de l'oeil. On parle de correction sphéro-cylindrique.

Quel que soit le type de cette lentille de correction, on pourra par ailleurs prévoir qu'elle présente en outre un revêtement antireflet (pour augmenter le contraste de la lettre « E » sur le fond) et/ou un revêtement teinté (pour modifier la couleur de la lettre « E » et du fond).

Quoi qu'il en soit, une fois la lentille de correction engagée dans la fente transversale 113, l'opticien redemande au patient s'il juge la lettre plus nette (par exemple s'il parvient à lire la lettre « E » représentée sur la cible 120).

S'il y parvient, l'opticien en déduit que le patient nécessite une paire de lunette de correction antifatigue.

Au contraire, s'il n'y parvient pas, l'opticien en déduit que le patient souffre de problèmes ophtalmiques autres que la simple fatigue, et il conseille donc au patient de se rendre chez un optométriste.

Les opérations sont ensuite mises en oeuvre de la même manière, sur l'oeil droit du patient.

Selon une variante de ce premier mode de réalisation, on pourrait calculer trois distances minimales d'accommodation pour chaque âge, dont :

- une distance normale d'accommodation $D_2=1/(18{,}5-1/3.\text{âge})$,
- une distance maximale d'accommodation $D_1=1/(15-0{,}25.\text{âge})$,
- une distance minimale d'accommodation $D_3=1/(24-0{,}4.\text{âge})$.

On obtiendrait ainsi trois échelles de graduations différentes, offrant à l'opticien une certaine liberté pour choisir le seuil à partir duquel il estimera qu'il y a fatigue ophtalmique.

Sur la figure 3, on a représenté le second mode de réalisation du dispositif de dépistage 200.

Dans ce mode de réalisation, le support 210 présente une forme quasi-identique à celle du support 110 représenté sur la figure 1.

Il présente ainsi une extrémité formant une partie d'appui 211 à plaquer contre le visage ou contre les lunettes du patient, ainsi qu'une glissière 212 dans laquelle coulisse la cible 220 et une fente transversale 213 pour accueillir une lentille de correction.

En l'espèce, le support 210 se distingue du support 110 représenté sur la figure 1 par les seules caractéristiques selon lesquelles il présente une longueur réduite et il comporte, parallèlement et à proximité de la fente transversale 213, entre la partie d'appui 211 et la cible 220, une seconde fente transversale permettant d'accueillir une lentille de changement d'échelle (dont la fonction sera précisée dans la suite de cet exposé).

La cible 220 présente quant à elle une forme identique à celle de la cible 120 représentée sur la figure 2.

Dans ce mode de réalisation, il est par ailleurs prévu trois graduations 230, 231, 232 distinctes.

Telle que représentée sur la figure 3, chaque graduation 230, 231, 232 est portée par un autocollant rectangulaire sur lequel sont seulement imprimés des âges.

Chaque autocollant est collé parallèlement à la glissière 212, à proximité de celle-ci et à distance des autres autocollants.

Les âges imprimés sur les autocollants ne sont pas les mêmes. Ainsi, quelle que soit sa position sur la glissière 212, la cible 220 pointe vers trois âges différents, selon que cet âge est lu sur l'un ou l'autre des autocollants.

On observe ainsi sur la figure 3 que l'une des graduations 231 fait apparaître des âges compris entre 16 et 30 ans, qu'une autre des graduations 230 fait apparaître des âges compris entre 31 et 46 ans, et que la dernière des graduations 232 fait apparaître des âges compris entre 37 et 49 ans.

Les trois graduations ne présentent donc pas la même échelle. Pour passer d'une graduation à l'autre, on utilise alors une lentille de changement d'échelle que l'on place à cet effet dans ladite seconde fente transversale, entre l'oeil du patient et la cible.

L'utilisation de trois graduations permet ainsi de réduire la longueur du support tubulaire 210, en ce sens que pour faire apparaître sur une seule et même graduation des âges compris entre 18 et 49 ans, il serait nécessaire de prévoir un support d'une longueur d'environ 50 centimètres.

L'utilisation de trois graduations permet également d'améliorer la précision des mesures. On observe en effet, en comparant les figures 1 et 3, que les âges 18 et 19 ans sont plus espacés sur la graduation 231 de la figure 3 que sur la graduation 130 de la figure 1, ce qui facilite la mise en place précise de la cible 220 en face de l'âge désiré.

Sur le premier marquage 230, chaque âge imprimé est situé à une distance de la partie d'appui 211 du support tubulaire 210 qui est sensiblement égale à la distance minimale d'accommodation qu'un individu est censé présenter à un âge donné s'il ne souffre d'aucun problème ophtalmique particulier. Cette distance notée $D_{âge}$ est calculée au moyen de la formule mathématique précitée et est fournie, en millimètres, sur la seconde colonne du tableau de la figure 6.

Sur les second et troisième marquages 231, 232, chaque âge imprimé est situé à une distance de la partie d'appui 211 du support tubulaire 210 qui est sensiblement égale à la distance minimale d'accommodation qu'un individu est censé présenter à un âge donné, s'il ne souffre d'aucun problème ophtalmique particulier et s'il porte une paire de lunettes équipée de lentilles dont les puissances sphériques sont soit de -5 dioptries (graduation 231), soit de 1,5 dioptrie (graduation 232).

Les distances calculées en fonction de l'âge du patient sont respectivement fournies, en millimètres, sur les quatrième et troisième colonnes du tableau de la figure 6.

On comprend alors que lorsqu'aucune lentille de mise à l'échelle n'est engagée dans la seconde fente transversale 214, la graduation à utiliser par l'opticien pour placer la cible 220 est la graduation 230 (en face de laquelle apparaît l'inscription « +0δ »). Lorsqu'une lentille de mise à l'échelle de -5 dioptries est engagée dans la seconde fente transversale 214, la graduation à utiliser par l'opticien pour placer la cible 220 est la graduation 231 (en face de laquelle apparaît l'inscription « -5δ »). Lorsqu'une lentille de mise à l'échelle de +1,5 dioptrie est engagée dans la seconde fente transversale 214, la graduation à utiliser par l'opticien pour placer la cible 220 est la graduation 232 (en face de laquelle apparaît l'inscription « +1.5δ »).

Le dépistage de l'éventuel état de fatigue ophtalmique du patient au moyen de ce dispositif de dépistage 200 est alors opéré sensiblement de la même manière qu'avec le dispositif de dépistage 100 représenté sur la figure 1.

La seule différence est que :

- si le patient a un âge compris entre 31 et 46 ans, l'opticien doit régler la position de la cible 220 en utilisant la graduation 230 ;

- si le patient a un âge compris entre 18 et 31 ans, l'opticien doit engager dans la seconde fente transversale 214 une lentille de mise à l'échelle de -5 dioptries et doit régler la position de la cible 220 en utilisant la graduation 231 ; et

- si le patient a un âge compris entre 47 et 49 ans, l'opticien doit engager dans la seconde fente transversale 214 une lentille de mise à l'échelle de +1,5 dioptries et doit régler la position de la cible 220 en utilisant la graduation 232.

Sur la figure 4, on a représenté le troisième mode de réalisation du dispositif de dépistage 300.

Dans ce mode de réalisation, le principe utilisé pour dépister un éventuel état de fatigue ophtalmique du patient est le même que celui utilisé avec le dispositif de dépistage 200 de la figure 3.

Le dispositif de dépistage 300 est en revanche binoculaire, et permet ainsi de détecter un éventuel état de fatigue du patient au choix simultanément ou successivement sur les deux yeux du patient.

Dans ce mode de réalisation, le support 310 se présente globalement sous la forme d'une paire de jumelles. Il présente plus précisément une forme globalement parallélépipédique allongée suivant un axe longitudinal A3, avec deux faces supérieure 310A et inférieure, deux faces latérales 310B, et deux faces d'extrémité appelées face frontale 310C et face arrière.

La face frontale 310C de ce support 310 est sensiblement plane. Elle présente une largeur d'environ 18 centimètres et une hauteur d'environ 7 centimètres. Elle est ouverte par deux fenêtres 315 rectangulaires permettant au patient de regarder avec ses deux yeux à l'intérieur du support 310. Ces fenêtres 315 sont ici obturées par des vitres transparentes, pour éviter l'infiltration de poussières à l'intérieur du support 310.

Pour permettre au patient de voir la cible 320, il est prévu à l'intérieur du support 310 des moyens d'éclairage, ici formés par des diodes électroluminescentes alimentées par des piles électriques.

La partie d'appui 311 de ce support 310, qui est destinée à être appliquée contre le visage ou contre les lunettes du patient, est alors formée par la face frontale 310C de ce support 310. Il est notamment prévu un renfoncement en creux dans l'arête inférieure de la face frontale 310C, qui forme un repose-nez.

La glissière 312 de ce support 310, dans laquelle coulisse la cible 320, est quant à elle formée par une fente longitudinale rectiligne qui s'étend sur une partie seulement de la longueur de la face supérieure 310A du support 310, parallèlement à l'axe longitudinal A3. Ici, cette glissière 312 ne débouche ni sur la face frontale 310C, ni sur la face arrière du support 310.

La cible 320 est identique à celle représentée sur la figure 2. Elle est positionnée dans le support 310 de telle manière qu'elle est simultanément visible par les deux yeux du patient au travers des fenêtres 315.

Dans ce mode de réalisation, il est prévu deux graduations 330, 331 distinctes, disposées de part et d'autre de la glissière 312.

Chaque graduation 330, 331 est portée par un autocollant rectangulaire sur lequel sont imprimés des âges. Ces deux autocollants sont alors collés parallèlement à la glissière 212, le long de celle-ci.

Comme dans le second mode de réalisation du dispositif de dépistage représenté sur la figure 3, les âges imprimés sur les deux autocollants ne sont pas représentés à la même échelle.

On observe ainsi sur la figure 4 que l'une des graduations 330 fait apparaître des âges compris entre 28 et 46 ans, tandis que l'autre des graduations 331 fait apparaître des âges compris entre 16 et 27 ans.

Il est alors prévu à l'intérieur du support 310 deux lentilles de mise à l'échelle (non visibles sur les figures), adaptées à être engagées en face des deux fenêtres 315 du support 310 ou à être dégagées de celles-ci, pour permettre d'utiliser l'une ou l'autre des deux graduations 330, 331.

Le dispositif de dépistage 300 comporte alors des moyens de manoeuvre manuelle de ces deux lentilles de mise à l'échelle, permettant de les engager ou de les dégager des fenêtres 315 du support 310, indépendamment l'une de l'autre.

Ici, ces moyens de manoeuvre sont formés par deux curseurs 314 qui sont situés sur la face supérieure 310A du support 310, en regard des deux fenêtres 315, et qui sont mobiles entre deux positions dans lesquelles ils pointent vers l'un ou l'autre de deux chiffres notés « 1 » et « 2 ».

On comprend alors que lorsque les curseurs 314 sont placés sur le chiffre « 1 », la graduation à utiliser par l'opticien pour positionner la cible 320 est la graduation 330 (en face de laquelle apparaît l'inscription « 1 »), et que lorsque les curseurs 314 sont placés sur le chiffre « 2 », la graduation à utiliser par l'opticien pour positionner la cible 320 est la graduation 331 (en face de laquelle apparaît l'inscription « 2 »).

Il est par ailleurs prévu à l'intérieur du support 310 deux masques (non visibles sur les figures), pour masquer l'une ou l'autre des fenêtres 315 lorsque l'opticien souhaite utiliser le dispositif de dépistage 300 successivement sur chaque oeil du patient et non simultanément sur les deux yeux du patient.

Le dispositif de dépistage 300 comporte alors des moyens de manoeuvre manuelle de ces deux masques, permettant de les engager ou de les dégager des fenêtres 315 du support 310, indépendamment l'un de l'autre.

Ici, ces moyens de manoeuvre sont formés par deux curseurs 316 qui sont respectivement situés sur les deux faces latérales 310B du support 310, à hauteur des deux fenêtres 315, et qui sont mobiles entre deux positions dans lesquelles ils pointent vers un cercle noir ou vers un disque noir.

On comprend alors que lorsque les curseurs 316 sont placés sur les disques noirs, les fenêtres 315 sont obturées, et que lorsque les curseurs 316 sont placés sur les cercles noirs, les fenêtres 315 sont dégagées pour laisser la cible 320 visible au travers des fenêtres 315.

Il est par ailleurs prévu à l'intérieur du support 310 deux lentilles de correction de 0,6 dioptrie (non visibles sur les figures) adaptées à être engagées en face des deux fenêtres 315 du support 310 ou à être dégagées de celles-ci.

Le dispositif de dépistage 300 comporte alors des moyens de manoeuvre manuelle de ces deux lentilles de correction, permettant de les engager ou de les dégager des fenêtres 315 du support 310, indépendamment l'une de l'autre.

Ici, ces moyens de manoeuvre sont formés par deux curseurs 313 qui sont situés sur la face supérieure 310A du support 310, en regard des deux fenêtres 315, et qui sont mobiles entre deux positions dans lesquelles ils pointent vers l'un ou l'autre de deux sigles notés « Ø » et « O ».

On comprend alors que lorsque les curseurs 313 sont placés sur le sigle « Ø », les lentilles de correction ne sont pas situées dans l'axe des fenêtres 315, tandis que lorsque les curseurs 313 sont placés sur le sigle « O », les lentilles de correction sont situées dans l'axe des fenêtres 315.

Le procédé d'utilisation de ce dispositif de dépistage 300 est le même que celui exposé supra, à la différence près qu'il est alors possible d'effectuer ce dépistage au choix, simultanément ou successivement sur les deux yeux du patient.

En cas de dépistage binoculaire simultané sur les deux yeux du patient, on pourra prévoir que les lentilles de correction présentent chacune une puissance prismatique non nulle, pour corriger une éventuelle fatigue prismatique des yeux.

Sur la figure 3, on a représenté le quatrième mode de réalisation du dispositif de dépistage 400.

Dans ce mode de réalisation, le dispositif de dépistage est formé par une simple tablette informatique qui comporte un châssis 402, un écran tactile 401 engagé dans le châssis 402, des moyens de détection 410 de la distance séparant l'écran tactile 402 du visage du patient, ainsi qu'une unité centrale qui est logée dans le châssis 402 et dans laquelle est mémorisé un logiciel de diagnostic.

Les moyens de détection 410, qui pourraient par exemple être formés par une simple caméra, sont ici formés par un capteur de distance à ultrasons.

Le dépistage de l'éventuel état de fatigue ophtalmique du patient au moyen de cette tablette 400 est alors opéré de la manière suivante.

Au préalable, le patient démarre le logiciel de diagnostic et y saisit son âge. Il s'équipe par ailleurs de ses lunettes de vue s'il en porte habituellement. Il écarte alors la tablette 400 de son visage jusqu'à la tenir à bout de bras.

Grâce aux moyens de détection 410, le logiciel de diagnostic acquiert alors la distance séparant l'écran tactile 401 des yeux du patient.

Le logiciel commande ensuite l'affichage sur l'écran tactile 401 d'un message indiquant au patient de rapprocher ou d'éloigner l'écran tactile 401 de ses yeux. L'objectif de cette opération est que la distance séparant l'écran tactile 401 des yeux du patient soit égale à la distance $D_{\text{âge}}$ (telle que définie dans le premier mode de réalisation de l'invention).

Le logiciel commande ensuite l'affichage sur l'écran tactile 401 de la lettre « E », ainsi que d'un message et de deux boutons pour permettre au patient de saisir le résultat de cet examen, afin que le logiciel acquière l'information selon laquelle le patient est parvenu ou non à lire la lettre affichée.

S'il y est parvenu, le logiciel commande l'affichage sur l'écran tactile 401 d'un message indiquant au patient qu'il ne nécessite pas de paire de lunettes antifatigue.

Au contraire, s'il n'y est pas parvenu, l'affichage sur l'écran tactile 401 d'un message indiquant au patient d'éloigner l'écran tactile 401 de ses yeux. Lorsque l'écran tactile 401 est suffisamment éloigné, le logiciel commande l'affichage sur l'écran tactile 401 d'une lettre « E » 420 agrandie afin de vérifier que seul un état de fatigue affecte les yeux du porteur (et que ce problème de vision n'est pas plus sérieux).

La taille de cette lettre « E » est calculée par rapport à la taille de la lettre « E » initialement affichée de telle manière que la lettre « E » apparaisse à taille constante pour le patient alors même que l'écran tactile 401 a été reculé. Un procédé de calcul permettant à la lettre « E » d'apparaître au patient avec un taille angulaire constate est présenté dans le document US7393102.

Tenant à nouveau la tablette 400 à bout de bras, le patient tente alors de lire la lettre « E » affichée sur l'écran tactile 401.

Le logiciel commande ensuite l'affichage sur l'écran tactile 401 d'un message et de deux boutons, pour permettre au patient de saisir le résultat de cet examen sur la tablette 400, afin que le logiciel acquière l'information selon laquelle le patient est parvenu ou non à lire la lettre affichée sur l'écran.

S'il y est parvenu, le logiciel commande l'affichage sur l'écran tactile 401 d'un message indiquant au patient qu'il nécessite une paire de lunettes antifatigue.

Au contraire, s'il n'y est pas parvenu, le logiciel commande l'affichage sur l'écran tactile 401 d'un message indiquant au patient de se rendre chez un optométriste pour des examens supplémentaires.

Le logiciel de diagnostic de la tablette 400, si cette dernière est connectée à un réseau informatique, peut alors commander l'envoi d'un message à un opticien fournissant le résultat de la mesure effectuée.

La présente invention n'est nullement limitée aux modes de réalisation décrits et représentés, mais l'homme du métier saura y apporter toute variante conforme à son esprit.

En particulier on pourrait prévoir que la taille de la lettre « E » affichée soir réglée en fonction également du moment de la journée auquel est effectué le dépistage, les yeux du patient étant naturellement plus fatigués le soir que le matin.

Encore en variante, on pourrait prévoir un dispositif de dépistage tel que celui représenté dans le premier mode de réalisation, mais dépourvu de fente transversale d'accueil d'une lentille de correction. Alors, lorsque le patient ne parviendrait pas à lire la lettre « E » imprimée sur la cible, l'opticien pourrait, non pas utiliser une lentille de correction, mais plutôt reculer la cible d'une distance donnée afin de faciliter la lecture de la lettre « E », cette distance correspondant par exemple à deux ans sur la graduation.

On pourrait par ailleurs prévoir que la partie d'appui du dispositif de dépistage soit adaptée à s'appuyer sur le visage du patient, à distance de ses yeux. Ainsi, le dispositif de dépistage s'appuiera de la même manière sur le visage du patient, c'est-à-dire dans la même position, que le patient porte ou non une paire de lunettes.

Selon une autre variante de l'invention non représentée sur les figures, on pourrait prévoir que la lentille de correction soit montée sur le support via un pivot, de manière à être escamotable. Elle serait ainsi mobile entre une position d'utilisation dans laquelle elle se trouverait entre la partie d'appui et la cible, et une position escamotée dans laquelle elle serait décalée angulairement de 90 degrés par rapport à sa position d'utilisation.

**Revendications**

1.  Procédé de dépistage d'un état de fatigue ophtalmique d'un individu, comportant des étapes :

   a1) de positionnement d'une cible (120 ; 220 ; 320 ; 420) relativement à l'individu de telle manière que la cible (120 ; 220 ; 320 ; 420) soit visible par au moins un des deux yeux de l'individu,

a2) d'appréciation par l'individu de la netteté de la cible (120 ; 220 ; 320 ; 420), et

a3) de déduction d'un état de fatigue ophtalmique de l'individu selon que la cible (120 ; 220 ; 320 ; 420) est vue par l'individu de manière nette ou floue,

le procédé comportant préalablement à l'étape a2), une étape a0) de réglage de la taille de la préalablement 320 ; l'étape et/ou une étape de a0) de réglage de la taille de la cible (120 ; 220 ; 320 ; 420) et/ou de la position de la cible (120 ; 220 ; 320 ; 420) relativement à l'individu, en fonction de l'âge de l'individu.

2. Procédé de dépistage selon la revendication précédente, dans lequel si, à l'étape a2), la cible (120 ; 220 ; 320 ; 420) est vue floue par l'individu, il est prévu suite à l'étape a3) des étapes :

b1) d'agrandissement de la taille de la cible (120 ; 220 ; 320 ; 420) et/ou d'éloignement de la cible (120; 220; 320 ; 420) relativement à l'individu d'une valeur prédéterminée,

b2) d'appréciation par l'individu de la netteté de la cible (120 ; 220 ; 320 ; 420), et

b3) si, à l'étape b2), l'individu voit la cible (120 ; 220 ; 320 ; 420) de manière plus nette qu'à l'étape a2), de déduction d'un besoin d'une correction antifatigue.

3. Procédé de dépistage selon l'une des revendications 1 et 2, dans lequel au cours de l'étape a0), seule la distance entre la cible (120 ; 220 ; 320 ; 420) et l'individu est réglée.

4. Procédé de dépistage selon les revendications 2 et 3, dans lequel l'étape b1) consiste à insérer au moins une lentille de correction entre l'oeil de l'individu et la cible (120 ; 220 ; 320 ; 420).

5. Procédé de dépistage selon la revendication précédente, dans lequel la lentille de correction présente une puissance sphérique non nulle et/ou une puissance cylindrique non nulle et/ou une puissance prismatique non nulle et/ou un revêtement antireflet et/ou un revêtement teinté, et/ou est formée par une lentille à profondeur de champ.

6. Procédé de dépistage selon l'une des revendications 1 et 2, dans lequel, au cours de l'étape a0), seule la taille de la cible (120 ; 220 ; 320 ; 420) est réglée.

7. Procédé de dépistage selon les revendications 2 et 6, dans lequel l'étape b1) consiste à agrandir la taille de la cible (120 ; 220 ; 320 ; 420).

8. Procédé de dépistage selon l'une des revendications précédentes, dans lequel, à l'étape a0), le réglage de la taille de la cible (120 ; 220 ; 320 ; 420) et/ou de la position de la cible (120 ; 220 ; 320 ; 420) relativement à l'individu est effectué en fonction également du moment de la journée auquel est effectué le dépistage, et/ou de l'origine géographique de l'individu et/ou de la netteté de la cible (120 ; 220 ; 320 ; 420) et/ou du contraste de la cible (120 ; 220 ; 320 ; 420).

9. Procédé de dépistage selon l'une des revendications précédentes, dans lequel, préalablement à l'étape a2), on équipe l'individu de son équipement de correction habituel.

10. Procédé de dépistage selon l'une des revendications précédentes, dans lequel les étapes a1) et a2) sont réalisées simultanément ou successivement sur les deux yeux de l'individu.

11. Procédé de dépistage selon l'une des revendications précédentes, dans lequel, suite à l'étape a3), il est prévu une étape c1) de mémorisation et de transmission à distance, via un réseau informatique, de l'état de fatigue ophtalmique déduit à l'étape a3).

12. Dispositif de dépistage (100; 200; 300) d'un état de fatigue ophtalmique d'un individu, comportant :

- un support (110 ; 210 ; 310) comportant une partie d'appui (111 ; 211 ; 311) à appliquer contre le visage ou contre les lunettes d'un individu et une glissière (112 ; 212 ; 312),

- une cible (120 ; 220 ; 320) montée mobile en coulissement sur la glissière (112 ; 212 ; 312) du support (110 ; 210 ; 310), et

- au moins une graduation (130 ; 230, 231, 232 ; 330, 331) située le long de la glissière (112 ; 212 ; 312) pour repérer la position de la cible (120 ; 220 ; 320) sur la glissière (112 ; 212 ; 312), qui fait apparaître des âges,

**caractérisé en ce que** le support (110 ; 210; 310) comporte des moyens de positionnement (113 ; 213 ; 313), situés entre la partie d'appui (111 ; 211 ; 311) et la cible (120 ; 220 ; 320), qui sont adaptés à accueillir au besoin une quelconque lentille de correction de manière que cette lentille de correction reste amovible, pour faciliter au besoin la vision de la cible.

13. Dispositif de dépistage (200 ; 300) selon la revendication 12, dans lequel il est prévu au moins deux graduations (230, 231, 232 ; 330, 331) faisant apparaître des âges et dans lequel il est prévu des moyens de positionnement (214 ; 314) d'une lentille de changement d'échelle, situés entre la partie d'appui (211 ; 311) et la cible (220 ; 320).

14. Dispositif de dépistage (100 ; 200 ; 300) selon l'une des revendications 12 à 13, dans lequel la cible (120 ; 220 ; 320) présente au moins une lettre ou une image.

**Patentansprüche**

1. Verfahren zur Prüfung eines Zustands ophthalmischer Ermüdung eines Individuums, die folgenden Schritte umfassend:

   a1) Positionierung eines Zielobjekts (120; 220; 320; 420) relativ zu dem Individuum, derart, dass das Zielobjekt (120; 220; 320; 420) für wenigstens eines der beiden Augen des Individuums sichtbar ist,
   a2) Beurteilung der Schärfe des Zielobjekts (120; 220; 320; 420) durch das Individuum, und
   a3) Ableitung eines Zustands ophthalmischer Ermüdung des Individuums je nachdem, ob das Zielobjekt (120; 220; 320; 420) von dem Individuum scharf oder unscharf gesehen wird,

   wobei das Verfahren vor dem Schritt a2) einen Schritt a0) der Einstellung der Größe des Zielobjekts (120; 220; 320; 420) und/oder der Position des Zielobjekts (120; 220; 320; 420) relativ zu dem Individuum in Abhängigkeit vom Alter des Individuums umfasst.

2. Verfahren zur Prüfung nach dem vorhergehenden Anspruch, wobei, falls im Schritt a2) das Zielobjekt (120; 220; 320; 420) von dem Individuum unscharf gesehen wird, im Anschluss an den Schritt a3) folgende Schritte vorgesehen sind:

   b1) Vergrößerung der Größe des Zielobjekts (120; 220; 320; 420) und/oder Entfernung des Zielobjekts (120; 220; 320; 420) relativ zu dem Individuum um einen vorbestimmten Wert,
   b2) Beurteilung der Schärfe des Zielobjekts (120; 220; 320; 420) durch das Individuum, und
   b3) falls im Schritt b2) das Individuum das Zielobjekt (120; 220; 320; 420) schärfer sieht als im Schritt a2), Ableitung eines Bedarfs an einer Korrektur gegen Ermüdung.

3. Verfahren zur Prüfung nach einem der Ansprüche 1 und 2, wobei im Schritt a0) nur der Abstand zwischen dem Zielobjekt (120; 220; 320; 420) und dem Individuum eingestellt wird.

4. Verfahren zur Prüfung nach den Ansprüchen 2 und 3, wobei der Schritt b1) darin besteht, wenigstens eine Korrekturlinse zwischen dem Auge des Individuums und dem Zielobjekt (120; 220; 320; 420) einzufügen.

5. Verfahren zur Prüfung nach dem vorhergehenden Anspruch, wobei die Korrekturlinse eine von null verschiedene sphärische Wirkung und/oder eine von null verschiedene zylindrische Wirkung und/oder eine von null verschiedene prismatische Wirkung und/oder eine Antireflexbeschichtung und/oder eine getönte Beschichtung aufweist und/oder von einer Feldtiefenlinse gebildet wird.

6. Verfahren zur Prüfung nach einem der Ansprüche 1 und 2, wobei im Schritt a0) nur die Größe des Zielobjekts (120; 220; 320; 420) eingestellt wird.

7. Verfahren zur Prüfung nach den Ansprüchen 2 und 6, wobei der Schritt b1) darin besteht, die Größe des Zielobjekts (120; 220; 320; 420) zu vergrößern.

8. Verfahren zur Prüfung nach einem der vorhergehenden Ansprüche, wobei im Schritt a0) die Einstellung der Größe des Zielobjekts (120; 220; 320; 420) und/oder der Position des Zielobjekts (120; 220; 320; 420) relativ zu dem Individuum auch in Abhängigkeit von der Tageszeit, zu der die Prüfung durchgeführt wird, und/oder der geographischen Herkunft des Individuums und/oder der Schärfe des Zielobjekts (120; 220; 320; 420) und/oder dem Kontrast des Zielobjekts (120; 220; 320; 420) vorgenommen wird.

9. Verfahren zur Prüfung nach einem der vorhergehenden Ansprüche, wobei vor dem Schritt a2) das Individuum mit seiner gewohnten Korrekturausrüstung ausgestattet wird.

10. Verfahren zur Prüfung nach einem der vorhergehenden Ansprüche, wobei die Schritte a1) und a2) gleichzeitig oder nacheinander an den beiden Augen des Individuums durchgeführt werden.

11. Verfahren zur Prüfung nach einem der vorhergehenden Ansprüche, wobei im Anschluss an den Schritt a3) ein Schritt c1) der Speicherung und der Fernübertragung, über ein Rechnernetz, des im Schritt a3) abgeleiteten Zustands ophthalmischer Ermüdung vorgesehen ist.

12. Vorrichtung zur Prüfung (100; 200; 300) eines Zustands ophthalmischer Ermüdung eines Individuums, welche aufweist:

   - einen Halter (110; 210; 310), der einen Stützteil (111; 211; 311), der an das Gesicht oder an die Brille eines Individuums anzusetzen ist, und eine Gleitführung (112; 212; 312) aufweist,
- ein Zielobjekt (120; 220; 320), das gleitbeweglich auf der Gleitführung (112; 212; 312) des Halters (110; 210; 310) angebracht ist, und
- wenigstens eine entlang der Gleitführung (112; 212; 312) angeordnete Skala (130; 230, 231, 232; 330, 331) zum Markieren der Position des Zielobjekts (120; 220; 320) auf der Gleitführung (112; 212; 312), auf der Altersangaben angebracht sind,

**dadurch gekennzeichnet, dass** der Halter (110; 210; 310) Positionierungsmittel (113; 213; 313) aufweist, die sich zwischen dem Stützteil (111; 211; 311) und dem Zielobjekt (120; 220; 320) befinden und die dafür ausgelegt sind, bei Bedarf eine beliebige Korrekturlinse aufzunehmen, derart, dass diese Korrekturlinse herausnehmbar bleibt, um bei Bedarf die Sichtbarkeit des Zielobjekts zu erleichtern.

13. Vorrichtung zur Prüfung (200; 300) nach Anspruch 12, bei der wenigstens zwei Skalen (230, 231, 232; 330, 331) vorgesehen sind, auf denen Altersangaben angebracht sind, und bei der Mittel zur Positionierung (214; 314) einer Linse zur Maßstabsänderung vorgesehen sind, die sich zwischen dem Stützteil (211; 311) und dem Zielobjekt (220; 320) befinden.

14. Vorrichtung zur Prüfung (100; 200; 300) nach einem der Ansprüche 12 bis 13, bei der das Zielobjekt (120; 220; 320) wenigstens einen Buchstaben oder ein Bild aufweist.

**Claims**

1. Method for screening for a state of ophthalmic fatigue of an individual, comprising steps:

   a1) of positioning a target (120; 220; 320; 420) relative to the individual in such a way that the target (120; 220; 320; 420) is visible by at least one of the two eyes of the individual;
   a2) of appraisal by the individual of the clearness of the target (120; 220; 320; 420); and
   a3) of deducing a state of ophthalmic fatigue of the individual depending on whether the target (120; 220; 320; 420) is seen clearly or hazily by the individual,

   the method comprising, prior to step a2), a step a0) of adjusting the size of the target (120; 220; 320; 420) and/or the position of the target (120; 220; 320; 420) relative to the individual, depending on the age of the individual.

2. Screening method according to the preceding claim, in which if, in step a2), the target (120; 220; 320; 420) is seen to be hazy by the individual, provision is made following step a3) for steps:

   b1) of enlarging the size of the target (120; 220; 320; 420) and/or of moving the target (120; 220; 320; 420) away from the individual by a preset distance;
   b2) of appraisal by the individual of the clearness of the target (120; 220; 320; 420); and
   b3) if, in step b2), the individual sees the target (120; 220; 320; 420) more clearly than in step a2), of deducing a need for an antifatigue correction.

3. Screening method according to either of Claims 1 and 2, in which, in step a0), only the distance between the target (120; 220; 320; 420) and the individual is adjusted.

4. Screening method according to Claims 2 and 3, in which step b1) consists in inserting at least one corrective lens between the eye of the individual and the target (120; 220; 320; 420).

5. Screening method according to the preceding claim, in which the corrective lens has a nonzero spherical power and/or a nonzero cylindrical power and/or a nonzero prismatic power and/or an antireflection coating and/or a tinted coating and/or is formed by a lens with a depth of field.

6. Screening method according to either of claims 1 and 2, in which, in step a0), only the size of the target (120; 220; 320; 420) is adjusted.

7. Screening method according to Claims 2 and 6, in which step b1) consists in enlarging the size of the target (120; 220; 320; 420).

8. Screening method according to one of the preceding claims, in which, in step a0), the size of the target (120; 220; 320; 420) and/or the position of the target (120; 220; 320; 420) relative to the individual are/is adjusted also depending on the time of day at which the screening is carried out and/or on the geographical origin of the individual and/or on the clearness of the target (120; 220; 320; 420) and/or on the contrast of the target (120; 220; 320; 420).

9. Screening method according to one of the preceding claims, in which, prior to step a2), the individual is equipped with his customary corrective equipment.

10. Screening method according to one of the preceding claims, in which steps a1) and a2) are carried out simultaneously or in succession on both eyes of the individual.

**11.** Screening method according to one of the preceding claims, in which, following step a3), provision is made for a step c1) of storing the state of ophthalmic fatigue deduced in step a3) in memory and of transmitting said state to a remote location.

**12.** Device (100; 200; 300) for screening for a state of ophthalmic fatigue of an individual, comprising:

- a carrier (110; 210; 310) comprising a bearing portion (111; 211; 311) to be applied against the face or against the spectacles of an individual and a slide rail (112 ; 212 ; 312);
- a target (120; 220; 320) slidably mounted in the slide rail (112; 212; 312) of the carrier (110; 210; 310); and
- at least one graduation (130; 230, 231, 232; 330, 331) located along the slide rail (112; 212; 312) for pinpointing the position of the target (120; 220; 320) in the slide rail (112; 212; 312), which indicates ages **characterized in that** the carrier (110; 210; 310) comprises positioning means (113; 213; 313), located between the bearing portion (111; 211; 311) and the target (120; 220; 320), that are suitable if required for receiving any corrective lens so that this corrective lens remains removable, in order if required to make it easier to see the target.

**13.** Screening device (200; 300) according to Claim 12, in which provision is made for at least two age-indicating graduations (230, 231, 232; 330, 331) and in which provision is made for means (214 ; 314) for positioning a scale-changing lens, said means being located between the bearing portion (211; 311) and the target (220; 320).

**14.** Screening device (100; 200; 300) according to either of Claims 12 and 13, in which the target (120; 220; 320) comprises at least one letter or image.

Fig.1

Fig.2

Fig.3

Fig.4

Fig.5

Fig.6

| Age | Dâge | +1.5 δ | -5 δ |
|---|---|---|---|
| 16 | 7.6 | 6.8 | 12.2 |
| 17 | 7.8 | 7.0 | 12.8 |
| 18 | 8.0 | 7.1 | 13.3 |
| 19 | 8.2 | 7.3 | 14.0 |
| 20 | 8.5 | 7.5 | 14.6 |
| 21 | 8.7 | 7.7 | 15.4 |
| 22 | 9.0 | 7.9 | 16.2 |
| 23 | 9.2 | 8.1 | 17.1 |
| 24 | 9.5 | 8.3 | 18.2 |
| 25 | 9.8 | 8.6 | 19.4 |
| 26 | 10.2 | 8.8 | 20.7 |
| 27 | 10.5 | 9.1 | 22.2 |
| 28 | 10.9 | 9.4 | 24.0 |
| 29 | 11.3 | 9.7 | 26.1 |
| 30 | 11.8 | 10.0 | 28.6 |
| 31 | 12.2 | 10.3 | 31.6 |
| 32 | 12.8 | 10.7 | 35.3 |
| 33 | 13.3 | 11.1 | 40.0 |
| 34 | 14.0 | 11.5 | 46.2 |
| 35 | 14.6 | 12.0 | 54.5 |
| 36 | 15.4 | 12.5 | 66.7 |
| 37 | 16.2 | 13.0 | 85.7 |
| 38 | 17.1 | 13.6 | 120.0 |
| 39 | 18.2 | 14.3 | 200.0 |
| 40 | 19.4 | 15.0 | 600.0 |
| 41 | 20.7 | 15.8 | |
| 42 | 22.2 | 16.7 | |
| 43 | 24.0 | 17.6 | |
| 44 | 26.1 | 18.8 | |
| 45 | 28.6 | 20.0 | |
| 46 | 31.6 | 21.4 | |
| 47 | 35.3 | 23.1 | |
| 48 | 40.0 | 25.0 | |
| 49 | 46.2 | 27.3 | |
| 50 | 54.5 | 30.0 | |

**RÉFÉRENCES CITÉES DANS LA DESCRIPTION**

**Documents brevets cités dans la description**

- US 7341350 B
- US 698833 A
- US 7393102 B

**Littérature non-brevet citée dans la description**

- La fatigue visuelle. INRS (Institut National de Recherche et de Sécurité), Mars 1992